(19) Europäisches Patentamt
European Patent Office
Office européen des brevets

(11) EP 4 039 302 B1

(12) FASCICULE DE BREVET EUROPEEN

(45) Date de publication et mention de la délivrance du brevet:
**09.10.2024 Bulletin 2024/41**

(21) Numéro de dépôt: **22150582.9**

(22) Date de dépôt: **07.01.2022**

(51) Classification Internationale des Brevets (IPC):
**A61M 16/00** (2006.01)    **A61M 16/10** (2006.01)
**A61M 16/06** (2006.01)    **A61M 16/20** (2006.01)

(52) Classification Coopérative des Brevets (CPC):
**A61M 16/024; A61M 16/0084; A61M 16/06;
A61M 16/1045; A61M 16/1075; A61M 16/204;
A61M 16/208;** A61M 16/1005; A61M 16/1055;
A61M 16/1065; A61M 16/122; A61M 2016/0027;
A61M 2016/0039; A61M 2202/0208;
A61M 2202/0283;      (Cont.)

(54) **APPAREIL DE FOURNITURE DE GAZ THÉRAPEUTIQUE À UN PATIENT AVEC CONTRÔLE DE LA PRESSION AU MASQUE**

GERÄT ZUR THERAPEUTISCHEN GASVERSORGUNG EINES PATIENTEN MIT STEUERUNG DES MASKENDRUCKS

DEVICE FOR SUPPLYING THERAPEUTIC GAS TO A PATIENT WITH CONTROL OF THE PRESSURE ON THE MASK

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **04.02.2021 FR 2101085**

(43) Date de publication de la demande:
**10.08.2022 Bulletin 2022/32**

(73) Titulaire: **L'Air Liquide, société anonyme pour l'Étude
et l'Exploitation des procédés Georges Claude
75007 Paris (FR)**

(72) Inventeur: **BOULANGER, Thierry
Newark, 19702 (US)**

(74) Mandataire: **Air Liquide
L'Air Liquide S.A.
Direction de la Propriété Intellectuelle
75, Quai d'Orsay
75321 Paris Cedex 07 (FR)**

(56) Documents cités:
**EP-A1- 3 698 833**      **WO-A1-2011/089491**
**WO-A1-2017/006253**

- **BORRELLO MIKE: "A feedback control approach to the estimation of patient airway and leak flow for Non-Invasive, Positive Pressure Ventilation (NPPV)", 2016 AMERICAN CONTROL CONFERENCE (ACC), AMERICAN AUTOMATIC CONTROL COUNCIL (AACC), 6 July 2016 (2016-07-06), pages 3982 - 3987, XP032933298, DOI: 10.1109/ACC.2016.7525535**

     **(Cont. page suivante)**

(52) Classification Coopérative des Brevets (CPC):
(Cont.)
A61M 2205/0216; A61M 2205/15; A61M 2205/18;
A61M 2205/3337; A61M 2205/3341;
A61M 2205/3344; A61M 2205/502; A61M 2205/505;
A61M 2205/583; A61M 2205/8206; A61M 2240/00

(52) Classification Coopérative des Brevets (CPC):
(Cont.)
A61M 2205/0216; A61M 2205/15; A61M 2205/18;

**Description**

L'invention concerne un appareil de délivrance de gaz pour fournir un gaz thérapeutique (i.e. gaz pur ou mélange gazeux) à un patient conscient dans différents lieux de soins, en particulier à l'hôpital, y compris dans le cadre d'inhalations de longue durée, par exemple de plusieurs heures, tout en minimisant les pertes de gaz.

Certaines thérapies nécessitent l'administration à des patients conscients de gaz thérapeutique formé d'un mélange de plusieurs constituants gazeux. Ainsi, il est connu d'utiliser un mélange équimolaire (50%/50%) de protoxyde d'azote ($N_2O$) et d'oxygène ($O_2$) pour amoindrir les états d'anxiété, produire un effet sédatif et/ou atténuer la douleur aigue. De même, il a été proposé d'utiliser un mélange d'argon et d'oxygène (60 vol.% Ar/40 vol.% $O_2$), inhalé avant et pendant, voire après, une procédure de thrombectomie mécanique, afin de traiter les accidents vasculaires cérébraux ou AVC.

Si l'administration du gaz par inhalation est généralement courte, c'est-à-dire typiquement de moins de 30 min, des inhalations plus longues, de l'ordre de 1h ou plus, sont parfois nécessaires.

De façon générale, l'inhalation du gaz thérapeutique (i.e. 1 ou plusieurs constituants) se fait via un masque respiratoire, typiquement un masque facial, i.e. naso-buccal, à un patient conscient et ce, de façon soit continue, soit intermittente, c'est-à-dire périodiquement.

La délivrance de gaz intermittente, c'est-à-dire non-continue, est souvent préférée. A cette fin, on utilise classiquement des dispositifs appelés « valves à la demande » (« VAD »).

Cependant, une VAD présente aussi des inconvénients. Ainsi, une VAD nécessite une pression négative minimale, relativement importante, pour s'ouvrir et fournir le débit de gaz au patient et, une fois ouverte, un effort inspiratoire conséquent de la part du patient est nécessaire pour inspirer le gaz dont il a besoin. Cet inconvénient peut être amplifié lorsque des tubulures de plusieurs mètres doivent être utilisées car elles vont engendrer une résistance supplémentaire à l'inspiration, laquelle est néfaste aux patients fragiles. De plus, la pression négative générée au niveau du masque, entraine souvent de l'air ambiant (i.e. défauts d'étanchéité) qui dilue le gaz thérapeutique et impacte négativement l'efficacité du gaz thérapeutique administré au patient.

Pour ces raisons, les VAD ne sont pas indiquées pour certains patients, en particulier les personnes fragiles (i.e. enfants en bas âge, personnes âgées...), et par ailleurs au traitement de certaines pathologies, en particulier lorsqu'elles affectent ces types de patients, par exemple les AVC qui frappent majoritairement des personnes âgées.

EP-A-3698833 propose un dispositif de délivrance automatique de gaz thérapeutique à un patient comprenant un passage de gaz avec un dispositif à vanne pour contrôler le débit de gaz alimentant un réservoir déformable, une unité de contrôle à microprocesseur pilotant le dispositif à vanne pour fixer ou ajuster le débit de gaz, des moyens de détermination de débit pour transmettre des mesures à l'unité de contrôle, un capteur de pression différentielle pour opérer des mesures de débit de gaz en aval du réservoir déformable et les fournir à l'unité de contrôle, et un masque respiratoire alimenté en gaz thérapeutique provenant du réservoir déformable. Ce dispositif permet de faciliter la respiration des patients faibles, notamment ceux atteints de BPCO. Si le dispositif, qui travaille de façon similaire à une VAD, permet de faciliter le travail respiratoire du patient, il ne permet pas de limiter la dilution du gaz thérapeutique en cas de défaut d'étanchéité au niveau du masque, i.e. en cas de fuites, car les tubulures de plusieurs mètres situées entre le dispositif et le patient génèrent une résistance à l'écoulement du flux gazeux et engendrent de fait une pression négative dans le masque.

Par ailleurs, M. Borello et al., « A feedback control approach to the estimation of the patient airway and leak flow for non-invasive positive pressure ventilation (NPPV), July 6-8, 2016, American Control Conférence (ACC) » et WO-A-2017/006253, proposent un algorithme permettant d'estimer les flux respiratoires et les fuites en ventilation non-invasive (NIV) chez des patients en soins critiques. L'algorithme est basé sur un contrôle simple, des mesures de pression proximale, le flux d'entrée du ventilateur médical fournissant le gaz d'assistance et un modèle non-linéaire du circuit de raccordement. Ces documents enseignent une mesure proximale de pression et une reconstitution du débit au masque via cette mesure de cette pression, une mesure de débit dans le ventilateur et un modèle du circuit.

Enfin, WO-A-2011/089491 enseigne un système de contrôle et régulation d'un flux de gaz fourni à un patient provenant d'un dispositif de génération de pression alimentant un circuit patient relié au patient. Un capteur de débit mesure le débit et un contrôleur détermine un ou des paramètres du flux respiratoire et utilise ces paramètres pour calculer un paramètre cible basé sur respiration-amplitude et un paramètre basé sur le temps de chaque cycle respiratoire du patient. Ce système vise un volume cible en prenant en compte la fuite intentionnelle du masque pour tenter de compenser le volume de gaz qui s'en échappe.

Ces solutions sont réservées à un type particulier de dispositifs d'administration de gaz car la reconstitution d'un signal utile pour leur fonctionnement repose sur une mesure de pression proximale issue d'un capteur de pression situé au niveau du masque patient. Un tel capteur alourdit le masque, ce qui peut créer des fuites inopinées. Comme ils opèrent en pression positive et donc empêchent toute introduction de gaz dans le masque patient, une mesure proximale de pression permet d'assurer une bonne ventilation du patient mais en créant un inconfort du patient. Ceci n'est toutefois pas applicable dans le cadre d'un système opérant à la manière d'une VAD, c'est-à-dire fonctionnant en pression « négative ».

Dans ce contexte, un problème est de proposer un appareil de délivrance de gaz thérapeutique, i.e. gaz pur ou mélange gazeux, à un patient permettant de limiter la consommation de gaz, c'est-à-dire fonctionnant de façon analogue à une VAD (avec pression « négative ») tout en assurant un effort inspiratoire minimal du patient pour garantir son confort respiratoire, y compris pendant des procédures longues (i.e. 1 à 2 h ou plus), et de limiter autant que possible la dilution du gaz thérapeutique en cas de fuites au masque et d'entrées indésirables d'air ambiant du fait de défauts d'étanchéité, c'est-à-dire un appareil qui n'engendre pas tout ou partie des problèmes rencontrés avec les VAD durant une délivrance de gaz intermittente, c'est-à-dire non-continue, à un patient ayant besoin d'inhaler un gaz thérapeutique dans le cadre d'un traitement médical.

Une solution selon l'invention concerne un appareil de délivrance de gaz thérapeutique à un patient comprenant :

- un passage de gaz interne en communication fluidique avec un réservoir déformable pour alimenter le réservoir déformable en gaz thérapeutique et pour récupérer du gaz thérapeutique provenant dudit réservoir déformable,
- un dispositif à vanne agencée sur le passage de gaz interne, en amont du réservoir déformable, pour contrôler le débit de gaz thérapeutique alimentant le réservoir déformable,
- une unité de contrôle à microprocesseur pilotant le dispositif à vanne pour fixer ou ajuster le débit de gaz traversant ledit dispositif à vanne,
- des moyens de détermination de débit agencés pour opérer une ou des mesures de pression ou de débit dans le passage de gaz interne, et transmettre ladite ou lesdites mesures à l'unité de contrôle,
- un capteur de pression configuré pour opérer une ou des mesures de pression de gaz ($P_{55}$) du gaz thérapeutique alimentant le réservoir déformable et fournir à l'unité de contrôle, la ou les mesures de pression de gaz ($P_{55}$), et
- un masque respiratoire en communication fluidique avec le passage de gaz interne et alimenté en gaz thérapeutique provenant du réservoir déformable,

et dans lequel :

- le capteur de pression est agencé sur le passage de gaz interne entre le dispositif à vanne et le réservoir déformable et

- l'unité de contrôle est configurée pour :

    i) déterminer le débit de gaz ($Q_{60}$) circulant dans le passage de gaz interne à partir de la ou des mesures de pression ou de débit fournies par les moyens de détermination de débit,
    ii) déterminer la pression de gaz ($P_{55}$) à partir de la ou des mesures de pression de gaz ($P_{55}$) provenant du capteur de pression,
    iii) déterminer les pertes de charges ($\Delta P$) existant en aval du capteur de pression à partir du débit de gaz ($Q_{60}$),
    iv) déterminer la pression ($P_M$) au niveau du masque respiratoire à partir de la pression de gaz ($P_{55}$) et des pertes de charges ($\Delta P$),
    v) et commander le dispositif à vanne pour ajuster le débit de gaz en fonction de ladite pression ($P_M$) déterminée au niveau du masque respiratoire de manière à ajuster la pression s'exerçant au niveau du masque respiratoire à une valeur égale à la pression atmosphérique.

Dans le cadre de l'invention :

- le terme « pression » est utilisé pour désigner de façon générale, une pression positive (> 0 bar), nulle (= 0 bar) ou négative (< 0 bar), c'est-à-dire une dépression.
- les pressions sont exprimées en bar ou mbar relatifs.
- le signe « - » devant une valeur de pression signifie que la pression est négative, c'est-à-dire qu'il s'agit d'une dépression (i.e. inférieure à la pression atmosphérique).
- le signe « + » devant une valeur de pression signifie que la pression est positive (i.e. supérieure à la pression atmosphérique).
- le terme « gaz thérapeutique » désigne un gaz à un ou plusieurs constituants ou composés gazeux, c'est-à-dire un gaz 'pur' ou un mélange gazeux.
- dans « unité de commande », le terme « unité » est équivalent aux termes « dispositifs », « appareils », « moyens », « système » ou analogues, et le terme « contrôle » est équivalent aux termes « commande », « pilotage », « traitement » ou similaire.

Selon le mode de réalisation considéré, l'appareil de délivrance de gaz thérapeutique de l'invention peut comprendre l'une ou plusieurs des caractéristiques suivantes :

- l'unité de contrôle (50) est configurée pour déterminer les pertes de charges ($\Delta P$) à partir d'une formule de calcul mémorisée, telle que : $\Delta P = R * Q_{60}^2$ avec :

    ▪ R est une constante.
    ▪ $Q_{60}$ est le débit de gaz ($Q_{60}$) circulant dans le passage de gaz interne.

- les moyens de détermination de débit sont agencés pour opérer une ou des mesures de pression ou de débit dans le passage de gaz interne, en aval du réservoir déformable.
- les moyens de détermination de débit comprennent un capteur de pression ou un capteur de débit, en particulier un capteur de pression différentielle ou un capteur de débit massique.

- il comprend des moyens de mémorisation pour mémoriser la formule de calcul des pertes de charges ($\Delta$P).
- le dispositif à vanne comprend une vanne proportionnelle.
- il comprend en outre au moins un dispositif anti-retour agencé dans le passage de gaz interne, en aval du réservoir déformable.
- au moins un dispositif anti-retour comprend un clapet anti-retour.
- le masque respiratoire est en communication fluidique avec le passage de gaz interne via une conduite de gaz flexible, i.e. tuyau flexible ou analogue.
- l'unité de contrôle comprend au moins une carte électronique portant au moins un microprocesseur.
- la conduite de gaz comprend un moyen de filtration, en particulier un filtre échangeur de chaleur et d'humidité.
- le passage de gaz interne comprend un ou plusieurs conduits, tuyaux ou analogue.
- les moyens de détermination de débit sont reliés électriquement à l'unité de contrôle.
- le capteur de pression est configuré pour fournir à l'unité de contrôle une ou des mesures de pression préférentiellement plusieurs mesures de pression successives, sous forme de valeurs numériques ou de signaux représentatifs de telles valeurs numériques, par exemple des signaux de tensions, lesquels valeurs ou signaux peuvent être traités tels quels ou convertis en valeurs numériques par l'unité de contrôle.
- le capteur de pression est configuré ou contrôlé pour opérer des mesures de pression à des intervalles de temps donnés, de préférence toutes les 20 msec ou moins, préférentiellement toutes les 10 msec ou moins, voire toutes les 5 msec ou moins.
- le capteur de pression est un capteur de pression relative comprenant un orifice de détection disposé dans le passage de gaz interne, et par ailleurs référencé à la pression atmosphérique (i.e. 0 mbar = 1 atm).
- le capteur de pression est un capteur de pression relative configuré pour fournir une ou des mesures de pression de gaz ($P_{55}$) correspondant chacune à une différence entre la pression absolue déterminée au niveau de l'orifice de détection (55a) et la pression atmosphérique.
- il comprend une source d'alimentation électrique de type cordon et prise d'alimentation au secteur (e.g. 110/220 V) et/ou une batterie interne, de préférence rechargeable.
- la source d'alimentation électrique alimente en courant électrique l'unité de contrôle et tous les autres composants de l'appareil présents (en fonction du mode de réalisation choisi) qui nécessitent de la puissance électrique pour fonctionner, par exemple un ou des composants de type écran d'affichage, LED, dispositif d'alarme sonore et/ou visuelle...

- il comprend un boitier externe rigide, par exemple en matériau polymère ou autre.
- l'unité de contrôle, au moins une partie du passage de gaz interne, le réservoir déformable, les moyens de détermination de débit et/ou le dispositif à vanne sont agencés dans le boitier.
- le réservoir déformable comprend un ballon flexible ou analogue.
- le réservoir déformable se déforme en fonction de la quantité et/ou la pression de gaz thérapeutique qu'il contient. Il peut donc adopter différents états, stades ou niveaux de remplissage, notamment un stade dit « plein », un stade dit « vide » (i.e. quantité résiduelle minimale de gaz) et des stades intermédiaires correspondant à un remplissage partiel du réservoir (i.e. entre les stades « plein » et « vide »).
- le réservoir déformable est en matériau flexible de type caoutchouc, silicone ou analogue, par exemple un caoutchouc-silicone LSR NuSil.
- l'unité de contrôle à microprocesseur comprend un ou plusieurs microprocesseurs, de préférence un (ou des) microcontrôleur.
- le (ou les) microprocesseur(s) met en oeuvre un ou plusieurs algorithmes.
- l'unité de contrôle comprend une ou plusieurs mémoires de stockage de données ou autres, par exemple une ou des formules de calcul, tables de référence....
- l'unité de contrôle à microprocesseur comprend une carte électronique portant le ou les microprocesseurs, de préférence un ou des microcontrôleurs.
- le réservoir déformable a un volume compris entre environ 0.1 et 3 L, mesuré au repos (i.e. pression interne égale à la pression atmosphérique).
- le réservoir déformable a une paroi d'épaisseur comprise entre 0.10 et 0.90 mm, typiquement entre 0.25 et 0.75 mm.
- l'appareil comprend en outre une (ou des) valve unidirectionnelle agencée dans le passage de gaz interne, en particulier en aval du réservoir déformable.
- l'appareil comprend en outre une interface homme-machine (IHM) comprenant un écran de visualisation d'informations, de préférence un écran tactile, et/ou une ou des touches ou boutons de sélection, notamment des touches virtuelles s'affichant sur l'écran tactile, et/ou un dispositif de mise en route, tel un bouton allumé/éteint ou « on/off », et/ou d'autres éléments.
- l'appareil comprend en outre un système d'alarme pour alerter l'utilisateur en cas de problème affectant l'appareil ou le gaz, par exemple un défaut de vanne ou de capteur, une mauvaise composition gazeuse (e.g. mélange hypoxique) ou autres. Le système d'alarme peut comprendre des moyens ou un dispositif d'émission de signaux sonores et/ou visuels.
- le masque respiratoire est un masque facial (i.e. naso-buccal) couvrant le nez et la bouche du patient, en utilisation, c'est-à-dire lorsqu'il est porté par ledit patient.

- il comprend en outre une source de gaz thérapeutique raccordée fluidiquement au passage interne de gaz pour alimenter ledit passage de gaz en gaz thérapeutique.
- la source de gaz thérapeutique comprend un ou plusieurs récipients de gaz, notamment des bouteilles.
- la source de gaz thérapeutique comprend un récipient de gaz contenant un mélange gazeux $O_2/N_2O$, de préférence un mélange équimolaire de $O_2/N_2O$ (i.e. 50 mol.%/50 mol.%).
- alternativement, la source de gaz thérapeutique comprend un récipient de gaz contenant un mélange gazeux $O_2$/argon, de préférence contenant de 35 à 45% vol.% $O_2$ et de 55 à 65% vol.% Ar, par exemple un mélange contenant de 38 à 43% vol.% $O_2$ et de 57 à 62% vol.% Ar, notamment un mélange binaire formé de 40% vol.% $O_2$ et 60% vol.% Ar.
- alternativement, la source de gaz thérapeutique comprend un premier récipient de gaz contenant de l'argon ou du $N_2O$, un second récipient de gaz contenant de l'oxygène ($O_2$) et un mélangeur de gaz alimenté en gaz par lesdits premier et second récipients de gaz, ledit mélangeur opérant un mélange des gaz provenant des premier et second récipients de gaz pour obtenir un mélange gazeux $O_2/N_2O$ ou $O_2$/argon.
- elle comprend un conduit d'amenée de pression, i.e. une liaison pneumatique, agencé entre le masque respiratoire et le capteur de pression de l'appareil de délivrance de gaz.

Ici également décrit est une méthode de traitement d'un patient par administration d'un gaz thérapeutique aux voies aériennes du patient utilisant un appareil de délivrance de gaz pour fournir le gaz thérapeutique, i.e. gaz pur ou mélange gazeux au patient conscient, en particulier à l'hôpital, notamment dans le cadre d'une inhalation de longue durée, par exemple de plusieurs heures, tout en minimisant les pertes de gaz.

Par exemple, la méthode de traitement peut comprendre l'administration par inhalation au patient d'un mélange de protoxyde d'azote ($N_2O$) et d'oxygène ($O_2$), tel équimolaire $N_2O/O_2$ (i.e. 50%/50%) destiné à traiter un état d'anxiété, produire un effet sédatif ou atténuer une douleur aigüe, ou d'un mélange d'argon et d'oxygène (e.g. 60 vol.% Ar/40 vol.% $O_2$), inhalé par exemple avant et pendant, voire après, une procédure de thrombectomie mécanique, afin de traiter les accidents vasculaires cérébraux (AVC), ou autre.

L'invention va maintenant être mieux comprise grâce à la description détaillée suivante, faite à titre illustratif mais non limitatif, en référence aux figures annexées parmi lesquelles :

Fig. 1 schématise une fourniture de gaz thérapeutique à un patient à l'aide d'un appareil de fourniture de gaz selon l'invention,

Fig. 2 schématise un mode de réalisation de l'architecture interne d'un appareil de fourniture de gaz selon l'invention,

Fig. 3 détaille les éléments agencés en aval du capteur de pression de l'appareil de délivrance de gaz de Fig. 2,

Fig. 4 schématise la relation pression/débit des éléments de Fig. 3.

Fig. 5 illustre le fonctionnement de l'unité de contrôle de l'appareil de délivrance de gaz de Fig. 2, en particulier les courbes de pression et de débit obtenues au fil du temps, et

Fig. 6 est une comparaison des performances d'un appareil de délivrance de gaz selon la présente invention et de plusieurs autres dispositifs.

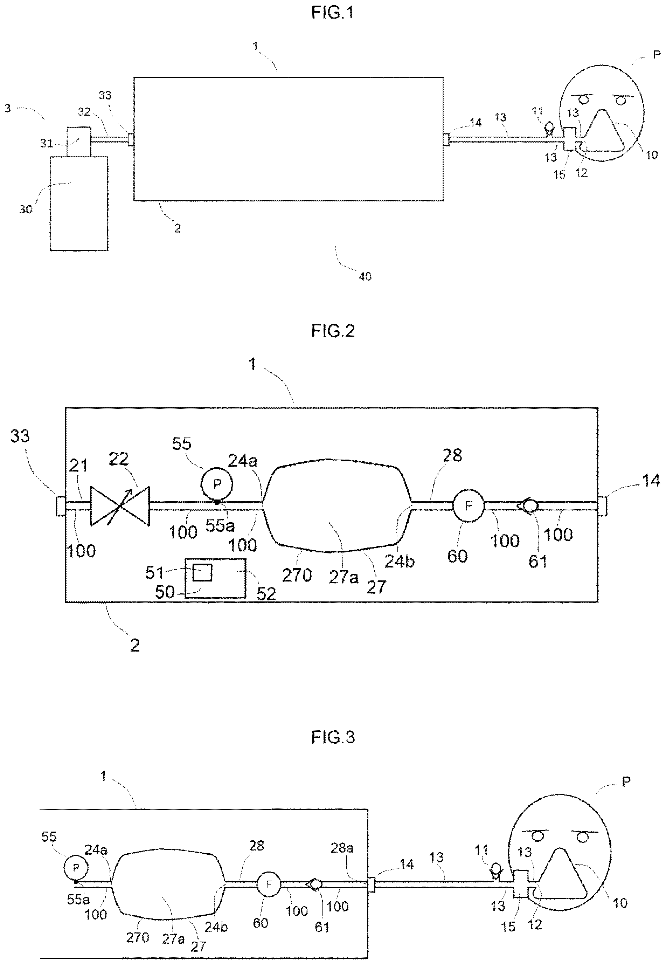

Fig. 1 est une représentation schématique de la fourniture de gaz thérapeutique à un patient mettant en oeuvre un appareil de fourniture de gaz 1 selon la présente invention, en particulier celui schématisé en Fig. 2.

Cet appareil 1 comprend un boitier externe 2 formant une carcasse rigide, par exemple en polymère, comprenant les composants internes, notamment un passage de gaz interne, un réservoir déformable, un dispositif à vanne et une unité de contrôle à microprocesseur comme expliqué ci-après.

Une source de gaz thérapeutique 3, telle qu'une bouteille de gaz 30 équipée d'une valve ou d'un robinet de distribution 31, fournit un gaz thérapeutique, c'est-à-dire un gaz ou mélange de gaz à l'appareil de délivrance de gaz 1 via un flexible de raccordement 32, connecté à un port d'entrée 33 de l'appareil 1.

Le gaz thérapeutique traverse l'appareil de délivrance de gaz 1, comme expliqué ci-après, pour ensuite être délivré à un patient P par le biais d'une conduite de gaz 13 flexible, tel un tuyau flexible en polymère, qui est raccordée fluidiquement à un port de sortie 14 de l'appareil 1. Le gaz est fourni au patient P via une interface ou masque respiratoire 10 alimenté par la conduite de gaz 13 flexible.

De préférence, l'interface ou masque respiratoire 10 est un masque facial, i.e. un masque naso-buccal, couvrant la bouche et le nez du patient. D'autres interfaces respiratoires peuvent bien entendu convenir et sont choisies en fonction du traitement à administrer au patient.

Le masque facial 10 présente ici un port d'inhalation 12 connecté fluidiquement à la conduite de gaz 13 qui achemine le gaz. Un filtre 15 est agencé dans la conduite de gaz 13.

Un port d'expiration 11 est agencé dans ladite conduite de gaz 13 en amont du filtre 15. Le port d'expiration 11 est fermé par un clapet anti-retour 11a qui contrôle le flux de gaz sortant en permettant l'évacuation à l'atmosphère des gaz expirés, lors des phases d'expiration du patient P, i.e. les gaz expirés riches en $CO_2$, et en empêchant par ailleurs l'entrée d'air ambiant dans la conduite de gaz 13, lorsque le patient inhale le gaz thérapeutique, c'est-à-dire pendant ses phases d'inspiration. Le clapet anti-retour 11a comprend une valve unidirectionnelle, tel un disque en silicone reposant sur une surface perforée, qui ne permet au gaz de ne passer que dans un sens, par exemple la valve unidirectionnelle de référence 97351 commercialisée par la société Qosina.

Le filtre 15, permettant de limiter l'exposition du patient à des particules fines ou à de potentielles bactéries ou autres microorganismes, est préférentiellement un filtre échangeur de chaleur et d'humidité. En effet, le mélange de gaz contenu dans la bouteille de gaz 30 est un gaz sec, c'est-à-dire dépourvu de vapeur d'eau. Une absence d'humidification des gaz inhalés pendant plusieurs heures peut assécher le système respiratoire du patient P en provoquant un inconfort, voire des lésions. Un filtre 15 échangeur de chaleur et d'humidité capte l'humidité présente dans les gaz expirés par le patient, et se réchauffe par la même occasion (du fait que les gaz expirés sont à la température corporelle, i.e. environ 37°C), lorsqu'ils traversent le filtre 15 pour être évacués à l'atmosphère ambiante, via le port d'expiration 11. Ce même filtre réchauffe et relargue ensuite cette humidité dans le gaz thérapeutique, durant l'inspiration suivante. De tels filtres sont bien connus et on peut utiliser par exemple un filtre Hydro-Therm® commercialisé par la société Intersurgical.

La source de gaz 3 contient un gaz thérapeutique sous pression, par exemple un mélange argon/oxygène, par exemple comprenant 60 vol.% d'argon et 40 vol.% d'oxygène, à une pression maximale de l'ordre de 250 bar. La valve ou robinet de distribution 31 est de préférence un robinet à régulateur de pression intégré ou RDI délivrant le gaz dans le flexible de raccordement 32 à une pression réduite, par exemple de l'ordre de 5 bar. Le régulateur de pression intégré 31 est de préférence protégé par un chapeau rigide (non représenté).

Alternativement, la source de gaz 3 peut comprendre plusieurs récipients de gaz 30, par exemple un premier récipient contenant de l'argon, un second récipient contenant de l'oxygène ($O_2$) et un mélangeur de gaz alimenté en gaz par lesdits premier et second récipients pour réaliser le mélange gazeux $O_2$/argon désiré qui est ensuite fourni à l'appareil de délivrance de gaz 1 selon la présente invention.

Bien entendu, l'appareil de délivrance de gaz 1 selon la présente invention peut aussi être utilisé pour fournir d'autres gaz thérapeutiques, tel qu'un $O_2$/$N_2O$ ou autre.

Fig. 2 schématise un mode de réalisation de l'architecture interne de l'appareil de délivrance de gaz 1 selon la présente invention qui fait partie de l'installation de fourniture de gaz 40 selon la présente invention, qui est schématisée en Fig.1.

L'appareil de délivrance de gaz 1 comprend une unité de contrôle 50 comprenant un (ou des) microprocesseur 51 porté par une carte électronique 52 servant à piloter un dispositif à vanne 22, de préférence une vanne proportionnelle, pour fixer ou ajuster le débit de gaz traversant ledit dispositif à vanne 22, comme expliqué ci-après.

L'unité de contrôle 50 comprend un (ou des) microprocesseur(s) 51, typiquement un (ou plusieurs) microcontrôleur(s), exécutant un (ou plusieurs) algorithme(s) qui reçoit et analyse les mesures fournies par différents capteurs, en particulier par un capteur de pression 55 et des moyens de détermination de débit 60 agencés dans le boitier 2.

Un passage de gaz interne 100, par exemple un conduit ou analogue, est arrangé dans le boitier 2 et s'étend entre un port ou orifice d'entrée 33 et un port ou orifice de sortie 14 de manière à convoyer le gaz thérapeutique depuis le port d'entrée 33 vers le port de sortie 14, et permettre ensuite son acheminement jusqu'au masque 10, via le conduit flexible 13.

Le dispositif à vanne 22, à savoir ici une vanne proportionnelle, est agencé dans le passage de gaz interne 100, de préférence dans la section amont 21 dudit passage de gaz interne 100. Il est contrôlé par le microcontrôleur 51 de l'unité de contrôle 50 pour modifier le débit de gaz thérapeutique traversant ledit dispositif à vanne 22 et circulant dans le lumen du passage de gaz interne 100 en direction du port ou orifice de sortie 14.

Différents types de vannes proportionnelles peuvent être utilisés en tant que dispositif à vanne 22, de préférence on choisit une vanne proportionnelle fonctionnant sur une large gamme de débits, par exemple la vanne référencée IMI FAS FLATPROP.

Un capteur de pression 55 est agencé dans le passage de gaz interne 100, en sortie du dispositif à vanne 22. Plus précisément, le capteur de pression 55 est agencé entre le dispositif à vanne 22 et le réservoir déformable 27 de manière à opérer des mesures de pression dans le passage de gaz interne 100 et en amont du réservoir déformable 27, comme expliqué ci-après.

Le capteur de pression 55 est configuré pour mesurer des pressions négatives, c'est-à-dire des pressions inférieures à la pression atmosphérique ou dépressions, et des pressions positives, c'est-à-dire des pressions supérieures à la pression atmosphérique ou surpressions, par exemple sur la plage de -10 mbar à +10 mbar.

Le capteur de pression 55 est ici un capteur de pression relative qui comprend un orifice de détection 55a disposé dans le passage de gaz interne 100, et qui est par ailleurs référencé aux conditions atmosphériques, i.e. à la pression atmosphérique (i.e. 0 mbar = 1 atm). Autrement dit, la pression retournée par le capteur de pression 55 est la différence entre la pression absolue régnant au niveau de son orifice de détection 55a, qui reflète la pression dans le passage de gaz interne 100, et la pression atmosphérique. On peut utiliser par exemple un capteur de pression relative de type True Stability® disponible auprès de la société Honeywell.

Le passage de gaz interne 100 achemine ensuite le gaz vers un réservoir déformable 27, en particulier un réservoir flexible, disposé en aval du capteur de pression 55, et en connexion fluidique avec le passage de gaz 100. Le réservoir déformable 27 comprend une paroi périphérique flexible 270 délimitant un volume interne 27a pour le gaz, formant une poche déformable pour le gaz thérapeutique. Au repos, le volume interne 27a est compris par exemple entre 0,1 et 1 L environ.

Le débit de gaz entre dans le volume interne 27a du réservoir déformable 27 par un orifice d'entrée de réservoir 24a, en communication fluidique avec le passage de gaz intérieur 100. De préférence, les propriétés du réservoir déformable 27 sont telles qu'il est hautement déformable. Par exemple, sa paroi périphérique 270 a une épaisseur comprise entre environ 0,25 et 0,75 mm et est formée d'un silicone flexible biocompatible, par exemple un matériau de type silicone de la série LSR commercialisée par NuSil.

Le gaz ressort du réservoir 27 par un orifice de sortie de réservoir 24b qui est relié fluidiquement à une section aval 28 du passage de gaz interne 100, se prolongeant jusqu'au port de sortie 14.

Des moyens de détermination de débit 60, à savoir un capteur de débit ou un capteur de pression, est agencé dans la section aval 28 du passage de gaz interne 100, afin de mesurer le débit ou la pression de gaz thérapeutique circulant dans ladite section aval 28. Les moyens de détermination de débit 60 peuvent être un capteur de débit massique ou un capteur de pression différentielle.

Les moyens de détermination de débit 60 sont connectés électriquement à l'unité de contrôle 50 et délivrent un (des) signal de débit ou de pression qui est traité par l'unité de contrôle 50, typiquement par le microprocesseur 51, préférentiellement un microcontrôleur.

De préférence, un débit volumétrique est obtenu après conversion du signal fourni par les moyens de détermination de débit 60 à l'aide d'une table de correspondance spécifique mémorisée dans une mémoire coopérant avec l'unité de contrôle 50.

Enfin, un (ou plusieurs) dispositif anti-retour 61, tel qu'un clapet anti-retour, est agencé dans le passage de gaz interne 100, à savoir en aval des moyens de détermination de débit 60 et en amont du port de sortie 14 du boitier 2, pour empêcher tout reflux de gaz. Ainsi, les gaz expirés par le patient P sont évacués uniquement par le port d'expiration 11 du masque 10 et ne peuvent pas retourner dans le réservoir 27.

Par ailleurs, une source d'alimentation électrique (non montrée) fournit du courant électrique à tous les composants fonctionnant avec de l'énergie électrique, tels que capteurs, unité de contrôle, vannes commandée, interface homme-machine (IHM), écran d'affichage numérique.... Elle peut être disposée dans le boitier 2, par exemple une batterie rechargeable ou comprend un cordon et une prise de raccordement au secteur (110/220V), et éventuellement un convertisseur de courant.

A des intervalles de temps successifs, par exemple toutes les 5 msec, le capteur de pression 55 envoie un signal de mesure de pression ($P_{55}$) à l'unité de contrôle 50. Ce signal $P_{55}$ reflète la pression régnant, à l'instant considéré, au niveau de l'orifice de détection 55a du capteur de pression 55, qui est disposé dans le passage de gaz interne 100.

De façon similaire, les moyens de détermination de débit 60 envoient, par exemple toutes les 5 msec, un signal de mesure de débit Q (ou de pression différentielle permettant de calculer le débit Q), à l'unité de contrôle 50. Le signal Q reflète le débit de gaz circulant dans le passage de gaz interne 100, et, par extension, la conduite de gaz 13.

L'unité de contrôle 50 traite alors ces signaux de pression et de débit afin de piloter la vanne proportionnelle 22 comme détaillé ci-après, pour ajuster le débit gazeux envoyé au réservoir flexible 27.

Le réservoir flexible 27 présente différents états de gonflage/dégonflage en fonction de la pression de gaz qui y règne, donc de la quantité de gaz qui y est introduite ou soutirée, comprenant au moins :

- un état dit « au repos », dans lequel le volume interne 27a, rempli de gaz, est à pression atmosphérique (i.e. 1 atm).
- un état dit « gonflé », dans lequel le volume interne 27a, rempli de gaz, est à une pression supérieure à la pression ambiante (c'est-à-dire> 1 atm).
- des états de « dégonflage partiel», dans lequel une partie du gaz contenu dans le réservoir en est sorti.

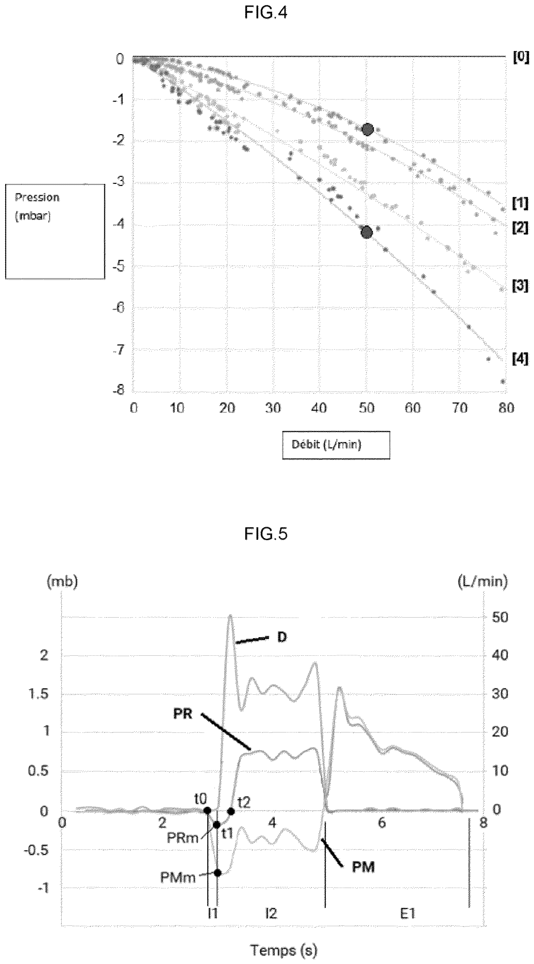

Fig. 3 détaille les éléments agencés en aval du capteur de pression 55 de l'appareil de délivrance de gaz 1, jusqu'au patient P et Fig. 4 schématise la relation pression/débit de ces différents éléments, c'est-à-dire la résistance (i.e. une pression) à l'écoulement d'un gaz (i.e. un débit) les traversant.

Un patient P est d'abord connecté fluidiquement aux éléments situés en aval du capteur de pression 55. L'orifice de détection 55a du capteur de pression 55 est alors à l'atmosphère, c'est-à-dire que la pression relative mesurée est égale à 0 mbar.

En réponse à une inhalation du patient P générant un débit donné Q, ce débit gazeux Q va circuler dans le passage de gaz interne 100, notamment au niveau de l'orifice de détection 55a du capteur de pression 55. La pression gazeuse existant dans le masque respiratoire 10 est alors la résultante des pertes de charge ($\Delta P$) des différents éléments situés en aval du capteur de pression 55.

La relation pression/débit est illustrée en Fig. 4 où :

- La courbe [0], qui est égale à 0 mbar relatif, c'est-à-dire à la pression ambiante, représente la pression mesurée par le capteur de pression 55.

- La courbe [1] représente les pertes de charges ΔP introduites entre le capteur de pression 55 et la terminaison aval 28a de la section aval 28 du passage de gaz interne 100. Par exemple, pour un débit Q de 50 L/min circulant dans cette portion, une dépression de -1.5mb relatif est nécessaire au niveau de la terminaison 28a.
- La courbe [2] représente les pertes de charge additionnelles engendrées par le port de sortie 14 de l'appareil 1.
- La courbe [3] représente les pertes de charge additionnelles engendrées par la portion de la conduite de gaz 13 située en aval du filtre 15.
- La courbe [4] représente les pertes de charge additionnelles engendrées par le filtre 15, incluant la portion aval de la conduite de gaz 13, jusqu'au port d'inhalation 12 du masque respiratoire 10.

Toutes ces pertes de charges additionnelles s'additionnent et cela conduit, pour un débit de 50 L/min, à une dépression de l'ordre de - 4mbar dans le masque respiratoire 10. En d'autres termes, le patient P devra fournir un effort inspiratoire conséquent afin de générer une telle pression négative.

Il apparait par ailleurs que la relation pression/débit entre le capteur de pression 55 et la pression dans le masque respiratoire, aussi appelée $P_{Masque}$ ou $P_M$, augmente lorsque le débit augmente. Elle suit une fonction polynomiale de type :

$$P_M = - R * Q^2$$

où :

- R est une constante rendant compte de la « résistance équivalente » des éléments situés entre le capteur de pression 55 et le masque respiratoire 10, et
- Q le débit traversant lesdits éléments.

Cette fonction polynomiale est aussi représentée en Fig. 4 sous la forme de courbes pleines.

On comprend qu'un tel niveau d'effort à fournir par le patient est inacceptable et que, dans le cadre de l'invention, il convient de réduire impérativement cette pression jusqu'à environ 0 mbar relatif, c'est-à-dire approximativement la pression atmosphérique.

Pour ce faire, selon l'invention, en connaissant les pertes de charges (ΔP) engendrées par les différents éléments en aval du capteur de pression 55, il est possible de reconstituer la pression $P_M$ régnant dans le masque respiratoire 10.

En particulier, considérant que la relation pression/débit « - R * Q² » est enregistrée dans l'unité de contrôle 50 à microcontrôleur 51, sous forme d'une (ou plusieurs) fonction polynomiale ou bien d'une table d'interpolation, la reconstitution de la pression au masque $P_M$ peut être réalisée par l'unité de contrôle 50.

A cette fin, l'unité de contrôle 50 opère le calcul suivant :

$$P_M = P_{55} - R * Q_{60}^2$$

où :

- $P_{55}$ est la pression mesurée par le capteur de pression 55, et
- $Q_{60}$ le débit circulant dans la section aval 28 du passage de gaz interne 100, mesuré par le capteur de débit 60.

Pour reprendre l'exemple de la courbe [4] (i.e. la pression dans le masque) et un débit $Q_{60}$ de 50L/min, on a alors : $P_M = P_{55} - 4$ (mbar)

Autrement dit, pour une pression relative $P_{55}$ nulle, comme illustré en Fig. 3, la pression résultante dans le masque est de -4 mbar environ.

L'unité de contrôle 50, en réponse à une mesure de débit de 50 L/min par les moyens de détermination de débit 60, peut piloter la vanne proportionnelle 22 pour minimiser la pression $P_M$, c'est-à-dire pressuriser le passage de gaz interne 100 de manière à ce qu'au niveau de l'orifice de détection 55a du capteur de pression 55, la pression soit positive, et dans ce cas précis, égale à environ 4 mbar environ.

En d'autres termes, l'unité de contrôle 50 pilote l'électrovanne 22 de manière à ce que, en réponse à une demande de débit du patient P, les pertes de charges des éléments situés en aval du capteur de pression 55, soient compensées.

Selon l'invention, l'unité de contrôle 50 peut déterminer, à un instant t, la pression $P_M$ dans le masque en procédant comme suit :

- Déterminer le débit $Q_{60}$ circulant dans la section aval 28 du passage de gaz interne 100, au moyen des moyens de détermination de débit 60.
- En déduire les pertes de charges (ΔP) induites par les éléments situés en aval du capteur de pression 55, via la fonction enregistrée : $R * Q_{60}^2$.
- Déterminer la pression $P_{55}$ au moyen du capteur de pression 55.
- En déduire la pression $P_M$ au masque.

La pression $P_M$ régnant dans le masque respiratoire 10 ainsi déterminée est donc une « pression reconstituée ».

Dans le cadre de l'invention, on cherche à minimiser cette pression $P_M$ « reconstituée » dans le masque afin de soulager l'effort inspiratoire du patient.

Comme illustré en Fig. 2, durant une thérapie avec administration de gaz thérapeutique, le patient P opère une succession d'inspirations et d'expirations pour inhaler le gaz thérapeutique, par exemple un mélange $O_2$/argon ou $N_2O/O_2$, et exhaler les gaz riches en $CO_2$ résultant des échanges pulmonaires.

Afin de faciliter la compréhension du fonctionnement de l'appareil 1, on considère que :

- la pression dans le volume interne 27a du réservoir 27 est égale à la pression atmosphérique, c'est-à-dire que le réservoir 27 est en position de repos.
- le réservoir 27 est rempli du gaz thérapeutique issu de la source de gaz 3.
- le patient entame une inspiration.

Lorsque le patient commence à inspirer, une légère dépression se produit au niveau du port d'inhalation 12 du masque 10. Cette dépression se propage en dans le conduit 13, le port de sortie 14 et la section aval 28 du passage de gaz interne 100.

Alors que la pression de gaz dans le volume interne 27a du réservoir 27 est égale à la pression atmosphérique (i.e. 1 atm), une pression différentielle positive apparaît alors et un débit gazeux, mesuré par les moyens de détermination de débit 60, peut s'établir depuis le réservoir 27 en direction du masque 10. Il s'ensuit que le volume interne 27a du réservoir 27 se vide alors et le réservoir 27 se dégonfle, créant à son tour une légère dépression dans le volume interne 27a.

Or, l'unité de contrôle 50 est configurée pour garantir qu'à tout moment, la pression régnant dans le masque 10 soit aussi proche que possible de la pression atmosphérique (i.e. 1 atm), soit 0 mbar relatif.

Pour ce faire, l'unité de contrôle 50 pilote la vanne proportionnelle 22 de manière à ce que le débit fourni par ladite vanne proportionnelle 22 soit proportionnel à la pression reconstituée $P_M$ dans le masque 10.

A cette fin, le microprocesseur 51 peut par exemple mettre en oeuvre un algorithme du type :

- Si « Pression masque » négative : Débit (L/min) = $\alpha$ * | P |
- Si « Pression masque » positive : Débit (L/min) = 0

où : $\alpha$ est une constante positive et | P | est la valeur absolue de la pression reconstituée $P_M$ dans le masque.

L'unité de contrôle 50 n'agit donc sur la vanne proportionnelle 22 que si la pression $P_M$ reconstituée dans le masque 10 est négative, c'est-à-dire que la vanne proportionnelle 22 est commandée ou reste en position fermée dès lors que la pression reconstituée dans le masque 10 devient positive.

En cas de pression reconstituée négative (i.e. dépression) dans le masque 10, traduisant une inspiration du patient P, la vanne proportionnelle 22 est commandée par l'unité de contrôle 50, tel que : Débit (L/min) = $\alpha$ * | P |.

Il apparait alors une proportionnalité entre le débit délivré par la vanne proportionnelle 22 et la pression négative reconstituée dans le masque 10. Plus la valeur de pression s'écarte de 0 mbar, plus le débit est élevé. A l'inverse, plus la valeur de pression se rapproche de 0 mbar, plus le débit est faible.

Bien entendu, un ou des algorithmes plus sophistiqués, tel qu'un contrôle par termes Proportionnel, Intégral et Dérivé (PID), pourraient être implémentés.

En outre, l'appareil de délivrance de gaz 1 peut comprend d'autres éléments, comme une interface homme-machine (IHM) avec écran de visualisation d'informations, de préférence un écran tactile, une ou des touches ou boutons de sélection, un dispositif de mise en route, tel un bouton allumé/éteint, un système d'alarme et/ou d'autres éléments.

Fig. 5 schématise le fonctionnement de l'unité de contrôle 50, en particulier de l'algorithme mis en oeuvre par le microprocesseur 51 de l'appareil de délivrance de gaz 1, en réponse à une inspiration du patient P.

L'inspiration du patient P se découpe en deux portions distinctes successives I1 et I2 avec I1 correspondant au tout début de l'inspiration. Ainsi, si t0 est l'instant même du début de l'inspiration du patient P, à cet instant la pression relative $P_R$ dans le réservoir 27 est nulle, c'est-à-dire à la pression atmosphérique (i.e. 1 atm).

Comme susmentionné, l'inspiration du patient crée alors une dépression dans le masque 10, qui est représentée par la courbe $P_M$. En réponse à cette dépression, l'unité de contrôle 50 va piloter la vanne proportionnelle 22 pour ajuster le débit de gaz thérapeutique afin de limiter la chute en pression dans le masque 10.

Or, comme dans tout système intégrant des éléments électromécaniques, il existe un temps de réponse intrinsèque, c'est-à-dire un délai, en réponse à la manifestation physique qu'est ici la dépression au masque 10.

Pendant cette phase I1, la pression dans le réservoir déformable 27 diminue, signe que ce dernier se dégonfle et qu'une quantité de gaz circule à travers les moyens de détermination de débit 60 et le clapet anti-retour 61 en direction du masque 10 et ce, afin de subvenir à la demande inspiratoire du patient P.

Cette diminution de pression dans le réservoir 27 atteint, en t1, une valeur minimale $P_{Rm}$ et, de façon similaire, il apparaît une pression minimale $P_{Mm}$ dans le masque 10.

Ce temps t1 correspond au moment où l'électrovanne proportionnelle 22 commence à s'ouvrir en réponse à la sollicitation de l'unité de contrôle 50 et donc à délivrer un débit D, marquant la transition en phase I2.

Dans ces conditions, le débit gazeux D va subvenir au besoin du patient P et dans le même temps remplir le réservoir 27 dont la pression $P_R$ va croitre jusqu'à redevenir nulle en t2, signe que le réservoir 27 est revenu dans son état de repos, c'est-à-dire pleinement rempli.

Cette augmentation de pression $P_R$ dans le réservoir 27 s'accompagne naturellement, dans le même temps, d'un accroissement de la pression $P_M$ reconstituée dans le masque 10, avoisinant ici environ -0.5 mbar.

La portion de la phase I2 ultérieure à t2 voit le réservoir 27 en situation de surgonflage car la pression $P_R$ est positive, ce qui est parfaitement normal.

En effet, comme décrit précédemment, l'unité de contrôle 50 pilote la vanne proportionnelle 22 de manière à ce que la pression $P_M$ reconstituée dans le masque 10 soit la plus proche possible de 0, et de fait, compense les pertes de charges des éléments situés en aval du capteur de pression 55, en réponse a un débit mesuré par les moyens de détermination de débit 60.

Enfin, la phase I2 laisse place à une phase expiratoire E1 où le patient expire à travers le port expiratoire 11. Cette expiration génère alors une pression reconstituée $P_M$ positive dans le masque 10 et l'unité de contrôle 50 commande alors la vanne proportionnelle 22 de manière à interrompre la délivrance de gaz, c'est-à-dire le débit. Dans le même temps, le réservoir 27, lui-même sous pression $P_R$ positive, se vide progressivement en suivant le profil de la pression $P_M$ régnant dans le masque 10.

Le réservoir 27 est indispensable au bon fonctionnement de l'appareil 1. En effet, s'il n'était pas présent, le gaz circulerait dans des éléments rigides, c'est-à-dire non déformables, comme le passage de gaz interne 100 et la conduite de gaz 13. Ainsi, lors de la phase I1, avant que la vanne proportionnelle 22 ne s'ouvre, la demande respiratoire du patient ne serait pas satisfaite, entrainant alors un inconfort respiratoire majeur pour le patient. De plus, le réservoir 27, tout au long de la phase inspiratoire, joue un rôle de tampon en atténuant l'effet des variations de la demande ventilatoire du patient P et de la réponse de l'unité de contrôle 50 et de la vanne proportionnelle 22, en réponse à ces variations.

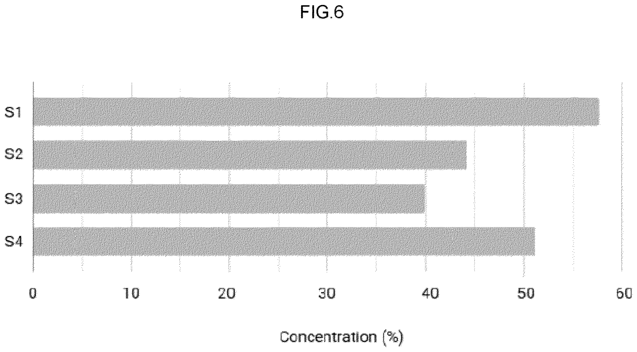

Fig. 6 compare les performances d'un appareil de délivrance de gaz 1 selon la présente invention et de plusieurs dispositifs de l'art antérieur, tels qu'un système à débit continu et une valve à la demande (VAD).

Afin d'obtenir des données représentatives, cette comparaison met en oeuvre un banc de test comprenant un « patient électronique », à savoir un dispositif mimant la respiration d'un patient, par exemple le simulateur respiratoire ASL 5000 disponible auprès de Ingmar Medical, permettant de simuler de façon répétable la respiration d'un patient.

Les différents dispositifs testés sont connectés au « patient électronique » au moyen d'un conduit d'acheminement de gaz à orifice calibré simulant une fuite au niveau du masque respiratoire.

La source de gaz thérapeutique fournit un mélange formé de 60% argon et de 40% oxygène (vol.%).

En fonction de la résistance de chacun des dispositifs, donc de la dépression générée par le « patient électronique », il est possible de mesurer la concentration en gaz thérapeutique inhalée par le patient sous l'effet de la dilution avec l'air ambiant.

Sur la Fig. 4 :

-   S1 est l'appareil de délivrance de gaz 1 de la présente invention, schématisé en Fig. 1 et Fig. 2 ;
-   S2 est une valve à la demande, par exemple la VAD de Ease II de GCE ;
-   S3 est un système à débit continu, par exemple celui disponible auprès de Intersurgical ; et
-   S4 est un appareil de délivrance de gaz analogue à celui de l'invention (i.e. S1) mais dans lequel le système de limitation de la dépression dans le masque a été supprimé (i.e. capteur, ligne d'amenée de pression...).

Les résultats obtenus montrent clairement les limitations des systèmes actuels.

Ainsi, sous l'effet de la fuite simulée, la concentration en argon inhalé par le patient P avoisine 40% (vol.%) pour le système à débit continu S3 et 45% pour la valve à la demande S2, soit respectivement une perte de 20% et 15% en volume d'argon, ce qui ne permet pas de garantir une efficacité du dispositif lors de la délivrance de gaz à un patient puisque la teneur en argon fournie au patient est très inférieure à celle attendue, i.e. 60% vol.

A l'inverse, l'appareil de délivrance de gaz 1 (S1) de l'invention permet de limiter fortement la dilution avec l'air ambiant, en maintenant, dans les mêmes conditions d'essai, une concentration de l'ordre de 57 vol.%, soit approximativement la teneur désirée (i.e. 60%), garantissant alors pleinement l'efficacité thérapeutique.

A titre comparatif, l'appareil de délivrance de gaz 1 dépourvu des moyens de limitation de la dépression dans le masque (S4), reste supérieur aux dispositifs existants (S2, S3) mais n'assure cependant qu'une concentration légèrement supérieure à 50 vol.%, ce qui est insuffisant pour garantir une efficacité du traitement à l'argon pour lequel une teneur efficace de 60 vol.% est désirée.

L'appareil de délivrance de gaz 1 selon l'invention répond donc en tout point aux besoins de confort patient et de minimisation de l'impact de fuite en terme de diminution de la concentration des gaz inhalés, assurant ainsi l'efficacité thérapeutique souhaitée. Ce niveau d'efficacité n'est possible qu'en associant, selon l'invention, un réservoir déformable 27 et à un asservissement du débit de gaz délivré à la pression reconstituée dans le masque 10, comme décrit ci-avant.

**Revendications**

1.   Appareil de délivrance de gaz thérapeutique (1) comprenant :

   - un passage de gaz interne (100) en communication fluidique avec un réservoir déformable (27) pour alimenter le réservoir déformable (27) en gaz thérapeutique et pour récupérer du gaz thérapeutique provenant dudit réservoir déformable (27),

- un dispositif à vanne (22) agencée sur le passage de gaz interne (100), en amont du réservoir déformable (27), pour contrôler le débit de gaz thérapeutique alimentant le réservoir déformable (27),
- une unité de contrôle (50) à microprocesseur (51) pilotant le dispositif à vanne (22) pour fixer ou ajuster le débit de gaz traversant ledit dispositif à vanne (22),
- des moyens de détermination de débit (60) agencés pour opérer une ou des mesures de pression ou de débit dans le passage de gaz interne (100), et transmettre ladite ou lesdites mesures à l'unité de contrôle (50),
- un capteur de pression (55) configuré pour opérer une ou des mesures de pression de gaz ($P_{55}$) du gaz thérapeutique alimentant le réservoir déformable (27) et fournir à l'unité de contrôle (50) la ou lesdites mesures de pression de gaz ($P_{55}$), et
- un masque respiratoire (10) en communication fluidique avec le passage de gaz interne (100) et alimenté en gaz thérapeutique provenant du réservoir déformable (27), dans lequel
- le capteur de pression (55) est agencé sur le passage de gaz interne (100) entre le dispositif à vanne (22) et le réservoir déformable (27) et
- l'unité de contrôle (50) est configurée pour :

a) déterminer le débit de gaz ($Q_{60}$) circulant dans le passage de gaz interne (100) à partir de la ou des mesures de pression ou de débit fournies par les moyens de détermination de débit (60),
b) déterminer la pression de gaz ($P_{55}$) à partir de la ou des mesures de pression de gaz ($P_{55}$) provenant du capteur de pression (55),
c) déterminer les pertes de charges ($\Delta P$) existant en aval du capteur de pression (55) à partir du débit de gaz ($Q_{60}$),
d) déterminer la pression ($P_M$) au niveau du masque respiratoire (10) à partir de la pression de gaz ($P_{55}$) et des pertes de charges ($\Delta P$),
e) et commander le dispositif à vanne (22) pour ajuster le débit de gaz en fonction de ladite pression ($P_M$) déterminée au niveau du masque respiratoire (10) de manière à ajuster la pression s'exerçant au niveau du masque respiratoire (10) à une valeur égale à la pression atmosphérique.

2. Appareil selon la revendication 1, où l'unité de contrôle (50) est configurée pour déterminer les pertes de charges ($\Delta P$) à partir d'une formule de calcul mémorisée, telle que : $\Delta P = R * Q_{60}^2$ avec :

- R est une constante rendant compte de la « résistance équivalente » des éléments situés entre le capteur de pression 55 et le masque respiratoire 10.
- $Q_{60}$ est le débit de gaz ($Q_{60}$) circulant dans le passage de gaz interne (100).

3. Appareil selon l'une des revendications précédentes, où les moyens de détermination de débit (60) sont agencés pour opérer une ou des mesures de pression ou de débit dans le passage de gaz interne (100), en aval du réservoir déformable (27).

4. Appareil selon l'une des revendications précédentes, où les moyens de détermination de débit (60) comprennent un capteur de pression ou un capteur de débit, en particulier un capteur de pression différentielle.

5. Appareil selon la revendication 2, où il comprend des moyens de mémorisation pour mémoriser la formule de calcul des pertes de charges ($\Delta P$).

6. Appareil selon la revendication 1, où le dispositif à vanne (22) comprend une vanne proportionnelle.

7. Appareil selon la revendication 1, où il comprend en outre un comprend au moins un dispositif anti-retour (61) agencé dans le passage de gaz interne (100), en aval du réservoir déformable (27).

8. Appareil selon la revendication 1, où le masque respiratoire (10) est en communication fluidique avec le passage de gaz interne (100) via une conduite de gaz (13) flexible.

9. Appareil selon la revendication 1, où l'unité de contrôle (50) comprend au moins une carte électronique portant au moins un microprocesseur.

10. Appareil selon la revendication 8, où la conduite de gaz (13) comprend un moyen de filtration (15), en particulier un filtre échangeur de chaleur et d'humidité.

11. Appareil selon la revendication 1, où l'unité de contrôle (50), au moins une partie du passage de gaz interne (100), le réservoir déformable (27), les moyens de détermination de débit (60) et le dispositif à vanne (22) sont agencés dans un boitier externe rigide (2).

12. Appareil selon la revendication 1, où le masque respiratoire est un masque facial.

13. Appareil selon la revendication 1, où le capteur de pression (55) est :

i) un capteur de pression relative comprenant un orifice de détection (55a) disposé dans le passage de gaz interne (100), et par ailleurs référencé à la pression atmosphérique (i.e. 0 mbar = 1 atm) et

ii) configuré pour fournir une ou des mesures de pression de gaz ($P_{55}$) correspondant chacune à une différence entre la pression absolue déterminée au niveau de l'orifice de détection (55a) et la pression atmosphérique.

14. Appareil selon la revendication 1, où une source de gaz thérapeutique (3) est raccordée fluidiquement au passage interne de gaz (100) pour alimenter ledit passage de gaz (100) en gaz thérapeutique, la source de gaz thérapeutique (3) comprenant un ou plusieurs récipients de gaz (30) comprenant :

- un récipient de gaz (30) contenant un mélange gazeux $O_2/N_2O$ ou un mélange gazeux $O_2$/argon, ou
- un premier récipient de gaz (30) contenant de l'argon ou du $N_2O$, un second récipient de gaz (30) contenant de l'oxygène ($O_2$) et un mélangeur de gaz alimenté en gaz par lesdits premier et second récipients de gaz (30) pour opérer un mélange des gaz provenant des premier et second récipients de gaz (30) et obtenir un mélange gazeux $O_2/N_2O$ ou $O_2$/argon.

**Patentansprüche**

1. Gerät zur Abgabe von therapeutischem Gas (1), umfassend:

- einen inneren Gasdurchgang (100), der in Fluidverbindung mit einem verformbaren Vorratsbehälter (27) steht, um dem verformbaren Vorratsbehälter (27) therapeutisches Gas zuzuführen und um aus dem verformbaren Vorratsbehälter (27) stammendes Gas zurückzugewinnen,
- eine Ventilvorrichtung (22), die an dem inneren Gasdurchgang (100), stromauf des verformbaren Vorratsbehälters (27), angeordnet ist, um den Volumenstrom des dem verformbaren Vorratsbehälter (27) zugeführten therapeutischen Gases zu steuern,
- eine Steuereinheit (50) mit Mikroprozessor (51), die die Ventilvorrichtung (22) ansteuert, um den die Ventilvorrichtung (22) durchquerenden Gasvolumenstrom festzulegen oder anzupassen,
- Volumenstrombestimmungsmittel (60), die dazu eingerichtet sind, eine oder mehrere Druck- oder Volumenstrommessungen in dem inneren Gasdurchgang (100) vorzunehmen und die Messung(en) an die Steuereinheit (50) zu übertragen,
- einen Drucksensor (55), der dazu ausgestaltet ist, eine oder mehrere Gasdruckmessungen ($P_{55}$) des dem verformbaren Vorratsbehälter (27) zugeführten therapeutischen Gases vorzunehmen und die Gasdruckmessung (en) ($P_{55}$) an die Steuereinheit (50) zu übermitteln, und
- eine Beatmungsmaske (10), die in Fluidverbindung mit dem inneren Gasdurchgang (100) steht und der aus dem verformbaren Vorratsbehälter (27) stammendes therapeutisches Gas zugeführt wird, wobei
- der Drucksensor (55) an dem inneren Gasdurchgang (100) zwischen der Ventilvorrichtung (22) und dem verformbaren Vorratsbehälter (27) angeordnet ist,
- die Steuereinheit (50) dazu ausgestaltet ist:

a) den Gasvolumenstrom ($Q_{60}$), der in dem inneren Gasdurchgang (100) zirkuliert, anhand der Druck- oder Volumenstrommessung(en), die von den Volumenstrombestimmungsmitteln (60) übermittelt werden, zu bestimmen,
b) den Gasdruck ($P_{55}$) anhand der Gasdruckmessung(en) ($P_{55}$), die von dem Drucksensor (55) stammen, zu bestimmen,
c) die Druckverluste ($\Delta P$), die stromab des Drucksensors (55) vorliegen, anhand des Gasvolumenstroms ($Q_{60}$) zu bestimmen,
d) den Druck ($P_M$) an der Beatmungsmaske (10) anhand des Gasdrucks ($P_{55}$) und der Druckverluste ($\Delta P$) zu bestimmen,
e) und die Ventilvorrichtung (22) zu steuern, um den Gasvolumenstrom in Abhängigkeit von dem an der Beatmungsmaske (10) bestimmten Druck ($P_M$) so anzupassen, dass der an der Beatmungsmaske (10) ausgeübte Druck auf einen Wert gleich dem atmosphärischen Druck angepasst wird.

2. Gerät nach Anspruch 1, wobei die Steuereinheit (50) dazu ausgestaltet ist, die Druckverluste ($\Delta P$) anhand einer gespeicherten Berechnungsformel zu bestimmen, so dass:

$$\Delta P = R * Q_{60}^2,$$

worin:

- R eine Konstante ist, die für den "äquivalenten Widerstand" der Elemente steht, die zwischen dem Drucksensor 55 und der Beatmungsmaske 10 gelegen sind,
- $Q_{60}$ der Gasvolumenstrom ($Q_{60}$) ist, der in dem inneren Gasdurchgang (100) zirkuliert.

**3.** Gerät nach einem der vorhergehenden Ansprüche, wobei die Volumenstrombestimmungsmittel (60) dazu eingerichtet sind, eine oder mehrere Druck- oder Volumenstrommessungen in dem inneren Gasdurchgang (100), stromab des verformbaren Vorratsbehälters (27), vorzunehmen.

**4.** Gerät nach einem der vorhergehenden Ansprüche, wobei die Volumenstrombestimmungsmittel (60) einen Drucksensor oder einen Volumenstromsensor umfassen, insbesondere einen Differenzdrucksensor.

**5.** Gerät nach Anspruch 2, wobei es Speichermittel umfasst, um die Berechnungsformel für die Druckverluste ($\Delta$P) zu speichern.

**6.** Gerät nach Anspruch 1, wobei die Ventilvorrichtung (22) ein Proportionalventil umfasst.

**7.** Gerät nach Anspruch 1, wobei es ferner mindestens eine Rückflussverhinderungsvorrichtung (61) umfasst, die in dem inneren Gasdurchgang (100), stromab des verformbaren Vorratsbehälters (27), angeordnet ist.

**8.** Gerät nach Anspruch 1, wobei die Beatmungsmaske (10) über eine flexible Gasleitung (13) in Fluidverbindung mit dem inneren Gasdurchgang (100) steht.

**9.** Gerät nach Anspruch 1, wobei die Steuereinheit (50) mindestens eine Elektronikkarte umfasst, die mindestens einen Mikroprozessor trägt.

**10.** Gerät nach Anspruch 8, wobei die Gasleitung (13) eine Filtereinrichtung (15) umfasst, insbesondere ein Wärme und Feuchtigkeit tauschendes Filter.

**11.** Gerät nach Anspruch 1, wobei die Steuereinheit (50), mindestens ein Teil des inneren Gasdurchgangs (100), der verformbare Vorratsbehälter (27), die Volumenstrombestimmungsmittel (60) und die Ventilvorrichtung (22) in einem starren Außengehäuse (2) angeordnet sind.

**12.** Gerät nach Anspruch 1, wobei die Beatmungsmaske eine Gesichtsmaske ist.

**13.** Gerät nach Anspruch 1, wobei der Drucksensor (55):

i) ein Relativdrucksensor ist, der eine Detektionsöffnung (55a) umfasst, die in dem inneren Gasdurchgang (100) angeordnet ist, und im Übrigen auf den atmosphärischen Druck bezogen ist (d. h. 0 mbar = 1 atm), und

ii) dazu ausgestaltet ist, eine oder mehrere Gasdruckmessungen ($P_{55}$) zu übermitteln, die jeweils einer Differenz zwischen dem an der Detektionsöffnung (55a) bestimmten absoluten Druck und dem atmosphärischen Druck entsprechen.

**14.** Gerät nach Anspruch 1, wobei eine Quelle für therapeutisches Gas (3) fluidisch an den inneren Gasdurchgang (100) angeschlossen ist, um dem Gasdurchgang (100) therapeutisches Gas zuzuführen, wobei die Quelle (3) für therapeutisches Gas einen oder mehrere Gasbehälter (30) umfasst, umfassend:

- einen Gasbehälter (30), der ein $O_2/N_2O$-Gasgemisch oder ein $O_2$/Argon-Gasgemisch enthält, oder
- einen ersten Gasbehälter (30), der Argon oder $N_2O$ enthält, einen zweiten Gasbehälter (30), der Sauerstoff ($O_2$) enthält, und einen Gasmischer, dem Gas von den ersten und zweiten Gasbehältern (30) zugeführt wird, um ein Mischen der aus den ersten und zweiten Gasbehältern (30) stammenden Gase vorzunehmen und ein $O_2/N_2O$- oder $Q_2$/Argon-Gasgemisch zu erhalten.

**Claims**

**1.** Apparatus (1) for delivering therapeutic gas, comprising:

- an internal gas passage (100) in fluidic communication with a deformable reservoir (27) in order to feed the deformable reservoir (27) with therapeutic gas and to recover therapeutic gas coming from said deformable reservoir (27),
- a valve device (22) arranged on the internal gas passage (100), upstream of the deformable reservoir (27), in order to control the flow rate of therapeutic gas feeding the deformable reservoir (27),
- a control unit (50) with microprocessor (51) controlling the valve device (22) in order to set or adjust the flow rate of gas passing through said valve device (22),
- flow rate determination means (60) designed to perform one or more measurements of pressure or flow rate in the internal gas passage (100) and to transmit said measurement(s) to the control unit (50),
- a pressure sensor (55) configured to perform one or more gas pressure measurements ($P_{55}$) on the therapeutic gas feeding the deformable reservoir (27) and to supply said gas pressure measurement (s) ($P_{55}$) to the control unit (50), and
- a breathing mask (10) in fluidic communication with the internal gas passage (100) and fed with therapeutic gas coming from the deformable reservoir (27), wherein

- the pressure sensor (55) is arranged on the internal gas passage (100) between the valve device (22) and the deformable reservoir (27), and
- the control unit (50) is configured to:

a) determine the flow rate of gas ($Q_{60}$) circulating in the internal gas passage (100) on the basis of the one or more pressure or flow rate measurements supplied by the flow rate determination means (60),

b) determine the gas pressure ($P_{55}$) on the basis of the one or more gas pressure measurements ($P_{55}$) coming from the pressure sensor (55),

c) determine the losses of head ($\Delta P$) downstream of the pressure sensor (55) on the basis of the gas flow rate ($Q_{60}$),

d) determine the pressure ($P_M$) at the breathing mask (10) on the basis of the gas pressure ($P_{55}$) and of the losses of head ($\Delta P$),

e) and control the valve device (22) to adjust the gas flow rate according to said pressure ($P_M$) determined at the breathing mask (10), in such a way as to adjust the pressure at the breathing mask (10) to a value equal to atmospheric pressure.

2. Apparatus according to Claim 1, where the control unit (50) is configured to determine the losses of head ($\Delta P$) using a stored calculation formula, namely: $\Delta P = R * Q_{60}^2$ where:

- R is a constant taking account of the "equivalent resistance" of the elements situated between the pressure sensor 55 and the breathing mask 10,
- $Q_{60}$ is the flow rate of gas ($Q_{60}$) circulating in the internal gas passage (100).

3. Apparatus according to one of the preceding claims, where the flow rate determination means (60) are arranged to perform one or more pressure or flow rate measurements in the internal gas passage (100), downstream of the deformable reservoir (27).

4. Apparatus according to one of the preceding claims, where the flow rate determination means (60) comprise a pressure sensor or a flow rate sensor, in particular a differential pressure sensor.

5. Apparatus according to Claim 2, where it comprises storage means for storing the formula for calculating the losses of head ($\Delta P$).

6. Apparatus according to Claim 1, where the valve device (22) comprises a proportional valve.

7. Apparatus according to Claim 1, where it further comprises at least one non-return device (61) arranged in the internal gas passage (100), downstream of the deformable reservoir (27).

8. Apparatus according to Claim 1, where the breathing mask (10) is in fluidic communication with the internal gas passage (100) via a flexible gas conduit (13).

9. Apparatus according to Claim 1, where the control unit (50) comprises at least one electronic board carrying at least one microprocessor.

10. Apparatus according to Claim 8, where the gas conduit (13) comprises a filtration means (15), in particular a heat and humidity exchange filter.

11. Apparatus according to Claim 1, where the control unit (50), at least part of the internal gas passage (100), the deformable reservoir (27), the flow rate determination means (60) and the valve device (22) are arranged in a rigid outer casing (2).

12. Apparatus according to Claim 1, where the breathing mask is a face mask.

13. Apparatus according to Claim 1, where the pressure sensor (55) is:

i) a relative pressure sensor comprising a detection orifice (55a) arranged in the internal gas passage (100), and moreover referenced to atmospheric pressure (i.e. 0 mbar = 1 atm), and
ii) configured to supply one or more gas pressure measurements ($P_{55}$), each corresponding to a difference between the absolute pressure determined at the detection orifice (55a) and the atmospheric pressure.

14. Apparatus according to Claim 1, where a source (3) of therapeutic gas is fluidically connected to the internal gas passage (100) in order to feed said gas passage (100) with therapeutic gas, the source (3) of therapeutic gas comprising one or more gas containers (30) comprising:

- a gas container (30) containing an $O_2/N_2O$ gas mixture or an $O_2$/argon gas mixture, or
- a first gas container (30) containing argon or $N_2O$, a second gas container (30) containing oxygen ($O_2$), and a gas mixer fed with gas by said first and second gas containers (30), so as to perform mixing of the gases coming from the first and second gas containers (30) and to obtain an $O_2/N_2O$ or $O_2$/argon gas mixture.

FIG.1

FIG.2

FIG.3

FIG.4

FIG.5

FIG.6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3698833 A
- WO 2017006253 A

- WO 2011089491 A

**Littérature non-brevet citée dans la description**

- **M. BORELLO et al.** A feedback control approach to the estimation of the patient airway and leak flow for non-invasive positive pressure ventilation (NPPV). *July 6-8, 2016, American Control Conférence (ACC),* 06 Juillet 2016